(19) Europäisches Patentamt
European Patent Office
Office européen des brevets

(11) Publication number : **0 342 850 B1**

(12) # EUROPEAN PATENT SPECIFICATION

(45) Date of publication of patent specification : **20.05.92 Bulletin 92/21**

(51) Int. Cl.$^5$ : **C07C 233/57**

(21) Application number : **89304698.7**

(22) Date of filing : **09.05.89**

(54) **Enantiomeric glutaramide diuretic agents.**

(30) Priority : **19.05.88 GB 8811873**

(43) Date of publication of application : **23.11.89 Bulletin 89/47**

(45) Publication of the grant of the patent : **20.05.92 Bulletin 92/21**

(84) Designated Contracting States : **AT BE CH DE ES FR GB GR IT LI LU NL SE**

(56) References cited : **EP-A- 0 274 234**

(73) Proprietor : **Pfizer Limited**
**Ramsgate Road**
**Sandwich Kent CT13 9NJ (GB)**
(84) **GB**

Proprietor : **PFIZER INC.**
**235 East 42nd Street**
**New York, N.Y. 10017 (US)**
(84) **BE CH DE ES FR GR IT LI LU NL SE AT**

(72) Inventor : **Danilewicz, John Christopher Dr.**
**44, Sandwich Road**
**Ash, Nr. Canterbury Kent (GB)**
Inventor : **Williams, Michael Trevelyan, Dr.**
**133, London Road**
**Deal Kent (GB)**

(74) Representative : **Moore, James William, Dr.**
**Pfizer Limited Ramsgate Road**
**Sandwich Kent CT13 9NJ (GB)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to certain spiro-substituted glutaramide derivatives which are diuretic agents having utility in a variety of therapeutic areas including the treatment of various cardiovascular disorders such as hypertension and heart failure.

According to the specification of our European patent application 0274234 we describe and claim a series of spiro-substituted glutaramide derivatives of the formula:

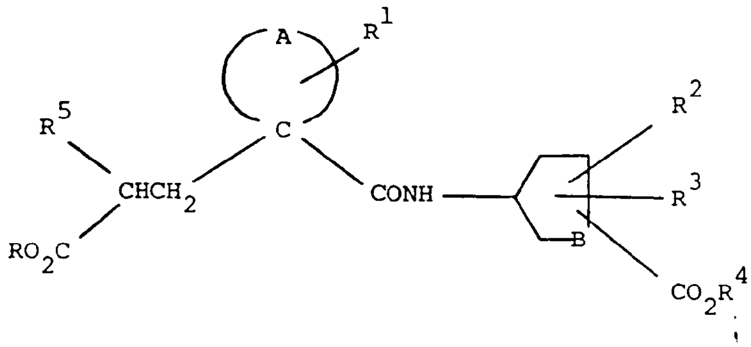

(I)

wherein

A completes a 4 to 7 membered carbocyclic ring which may be saturated or mono-unsaturated and which may optionally be fused to a further saturated or unsaturated 5 or 6 membered carbocyclic ring;

B is $(CH_2)_m$ wherein m is an integer of from 1 to 3; each of R and $R^4$ is independently H, $C_1$-$C_6$ alkyl, benzyl or an alternative biolabile ester-forming group;

$R^1$ is H or $C_1$-$C_4$ alkyl;

$R^2$ and $R^3$ are each independently H, OH, $C_1$-$C_4$ alkyl or $C_1$-$C_4$ alkoxy;

and

$R^5$ is $C_1$-$C_6$ alkyl, $C_2$-$C_6$ alkenyl, $C_2$-$C_6$ alkynyl, aryl($C_2$-$C_6$ alkynyl), $C_3$-$C_7$ cycloalkyl, $C_3$-$C_7$ cycloalkenyl, $C_1$-$C_6$ alkoxy, $-NR^6R^7$, $-NR^8COR^9$, $-NR^8SO_2R^9$ or a saturated heterocyclic group;

or $C_1$-$C_6$ alkyl substituted by one or more substituents chosen from halo, hydroxy, $C_1$-$C_6$ alkoxy, $C_2$-$C_6$ hydroxyalkoxy, $C_1$-$C_6$ alkoxy($C_1$-$C_6$ alkoxy), $C_3$-$C_7$ cycloalkyl, $C_3$-$C_7$ cycloalkenyl, aryl, aryloxy, aryloxy($C_1$-$C_4$ alkoxy), heterocyclyl, heterocyclyloxy, $-NR^6R^7$, $-NR^8COR^9$, $-NR^8SO_2R^9$, $-CONR^6R^7$, -SH, $-S(O)_pR^{10}$, $-COR^{11}$ or $-CO_2R^{12}$;

wherein

$R^6$ and $R^7$ are each independently H, $C_1$-$C_4$ alkyl, $C_3$-$C_7$ cycloalkyl (optionally substituted by hydroxy or $C_1$-$C_4$ alkoxy), aryl, aryl($C_1$-$C_4$ alkyl), $C_2$-$C_6$ alkoxyalkyl, or heterocyclyl; or the two groups $R^6$ and $R^7$ are taken together with the nitrogen to which they are attached to form a pyrrolidinyl, piperidino, morpholino, piperazinyl or N-($C_1$-$C_4$ alkyl)-piperazinyl group;

$R^8$ is H or $C_1$-$C_4$ alkyl;

$R^9$ is $C_1$-$C_4$ alkyl, $CF_3$, aryl, aryl($C_1$-$C_4$ alkyl), aryl($C_1$-$C_4$ alkoxy), heterocycyl, $C_1$-$C_4$ alkoxy or $NR^6R^7$ wherein $R^6$ and $R^7$ are as previously defined;

$R^{10}$ is $C_1$-$C_4$ alkyl, aryl, heterocyclyl or $NR^6R^7$ wherein $R^6$ and $R^7$ are as previously defined;

$R^{11}$ is $C_1$-$C_4$ alkyl, $C_3$-$C_7$ cycloalkyl, aryl or heterocyclyl;

$R^{12}$ is H or $C_1$-$C_4$ alkyl;

and

p is 0, 1 or 2;

and pharmaceutically acceptable salts thereof and bioprecursors therefor.

The compounds are inhibitors of the zinc-dependent, neutral endopeptidase E.C.3.4.24.11. This enzyme is involved in the breakdown of several peptide hormones, including atrial natriuretic factor (ANF), which is secreted by the heart and which has potent vasodilatory, diuretic and natriuretic activity. Thus, by inhibiting the neutral endopeptidase E.C.3.4.24.11, the compounds can potentiate the biological effects of ANF and, in particular, the compounds are diuretic agents having utility in the treatment of a number of disorders, including

hypertension, heart failure, angina, renal insufficiency, premenstrual syndrome, cyclical oedema, Menières disease, hyperaldosteroneism (primary and secondary) and hypercalciuria. In addition, because of their ability to potentiate the effects of ANF the compounds have utility in the treatment of glaucoma. As a further result of their ability to inhibit the neutral endopeptidase E.C.3.4.24.11 the compounds of the invention may have activity in other therapeutic areas including for example the treatment of asthma, inflammation, pain, epilepsy, affective disorders, dementia and geriatric confusion, obesity and gastrointestinal disorders (especially diarrhoae and irritable bowel syndrome), the modulation of gastric acid secretion and the treatment of hyperreninaemia.

Particularly preferred compounds according to European patent application 0274234 are:

cis-4-{1-[2-carboxy-3-(2-methoxyethoxy)propyl]-1-cyclopentanecarboxamido}-1-cyclohexanecarboxylic acid and biolabile ester derivatives thereof, including in particular the indanyl ester:

cis-4-{1-[2-(5-indanyloxycarbonyl)-3-(2-methoxyethoxy)propyl]-1-cyclopentanecarboxamido}-1-cyclohexanecarboxylic acid.

It will be noted that the two above compounds have an asymmetric carbon atom and therefore exist as R and S enantiomeric forms. We have now separated the isomers and unexpectedly discovered that the biological activity resides exclusively in the (+) enantiomer of diacid (II) (R = $R^4$ = H), to which we have assigned the S configuration. The R enantiomer is virtually inactive. Thus the present invention provides S enantiomeric compounds of the formula:-

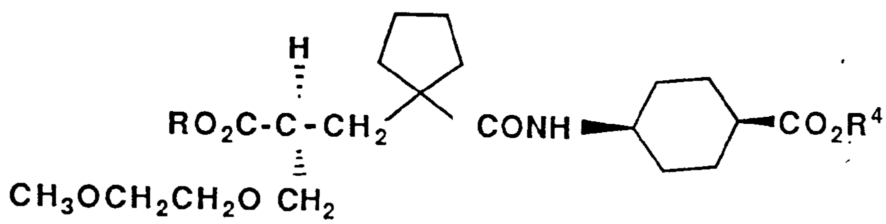

(II)

wherein each of R and $R^4$ is H or one of R and $R^4$ is H and the other is a biolabile ester group, said enantiomer being substantially free of the R enantiomer.

By substantially free of the R enantiomer is meant that the compounds of formula (II) contain less than 10%, and preferably less than 5% of the R enantiomer.

The term biolabile ester-forming group is well understood in the art as meaning a group which provides an ester which can be readily cleaved in the body to liberate the corresponding diacid of formula (II) wherein R and $R^4$ are both H. Examples of such esters include, in particular,

ethyl
benzyl
1-(2,2-diethylbutyryloxy)ethyl
2-ethylpropionyloxymethyl
1-(2-ethylpropionyloxy)ethyl
1-(2,4-dimethylbenzoyloxy)ethyl
α-benzoyloxybenzyl
1-(benzoyloxy)ethyl
2-methyl-1-propionyloxy-1-propyl
2,4,6-trimethylbenzoyloxymethyl
1-(2,4,6-trimethylbenzoyloxy)ethyl
pivaloyloxymethyl
phenethyl
phenpropyl
2,2,2-trifluoroethyl
1- or 2-naphthyl
2,4-dimethylphenyl
4-t-butylphenyl

and 5-indanyl.

Of these a particular preferred biolabile ester-forming group is 5-indanyl.

Thus particularly preferred individual compounds according to the invention are:

2(S)-cis-4-{1-[2-carboxy-3-(2-methoxyethoxy)propyl]-1-cyclopentanecarboxamido}- 1-cyclohexanecarboxylic acid and

2(S)-cis-4-{1-[2-(5-indanyloxycarbonyl)-3-(2-methoxyethoxy)propyl]-1-cyclopentanecarboxamido}-1-cyclohexanecarboxylic acid

The compounds of formula (II) are prepared generally following the synthetic procedures already disclosed in European patent application 0274234 but incorporating a resolution step at some convenient point in the synthetic sequence. Such resolution may be achieved by known techniques such as by fractional crystallisation of a salt formed with an optically active base or by chromatographic resolution of a diastereoisomeric derivative, such as, for example, an ester formed by reaction with an optically active alcohol.

Thus, in one process for preparation of the bis-acid of formula (II) wherein R and $R^4$ are both H, the following synthetic procedure may be employed wherein (NAE) indicates the N-acetyl-(1R,2S)-ephedrine ester:-

Scheme 1

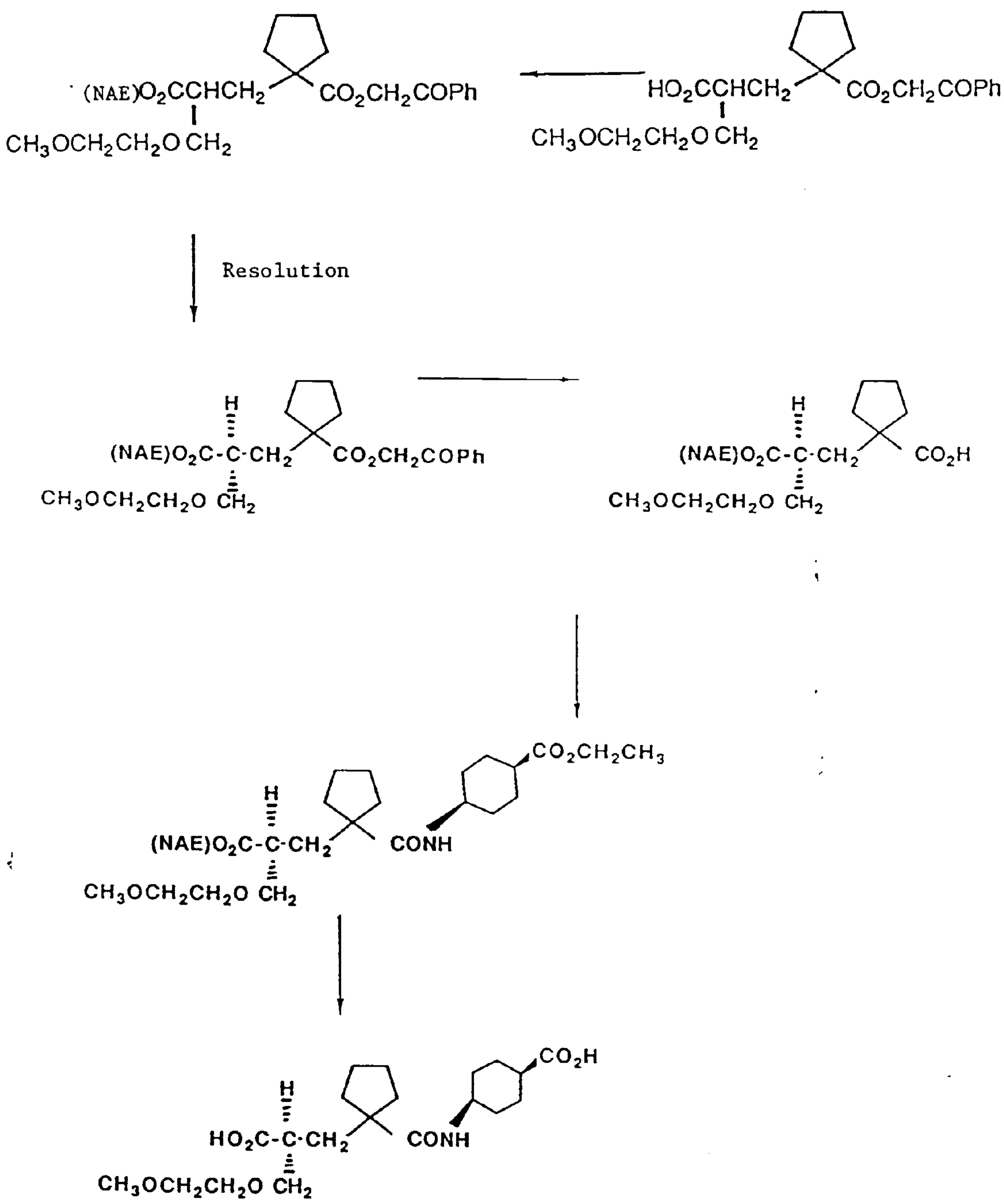


( III ) ( S ) ( + ) enantiomer

In this process N-acetyl-(1R,2S)-ephedrine is coupled to 2-(2-methoxyethoxymethyl)-3-[1-(phenacyloxycarbonyl)cyclopentyl]propanoic acid (prepared as described in European patent application no. 0274234) using, for example, a carbodiimide coupling reaction. Resolution of the resulting diester product may be achieved by chromatography on silica. The required separated diastereoisomer is treated with zinc in glacial acetic acid to remove the phenacyl ester group and the product is coupled with cis-4-aminocyclohexane carboxylic acid ethyl ester, again using a carbodiimide coupling reaction. The ester groups are finally removed by catalytic hydrogenation followed by mild alkaline hydrolysis to yield the dicarboxylic acid (III) as its dextrorotatory S enantiomer.

In a further process for preparing the compound of formula (II) wherein R is 5-indanyl and $R^4$ is H the following synthetic sequence may be followed:-

Scheme 2

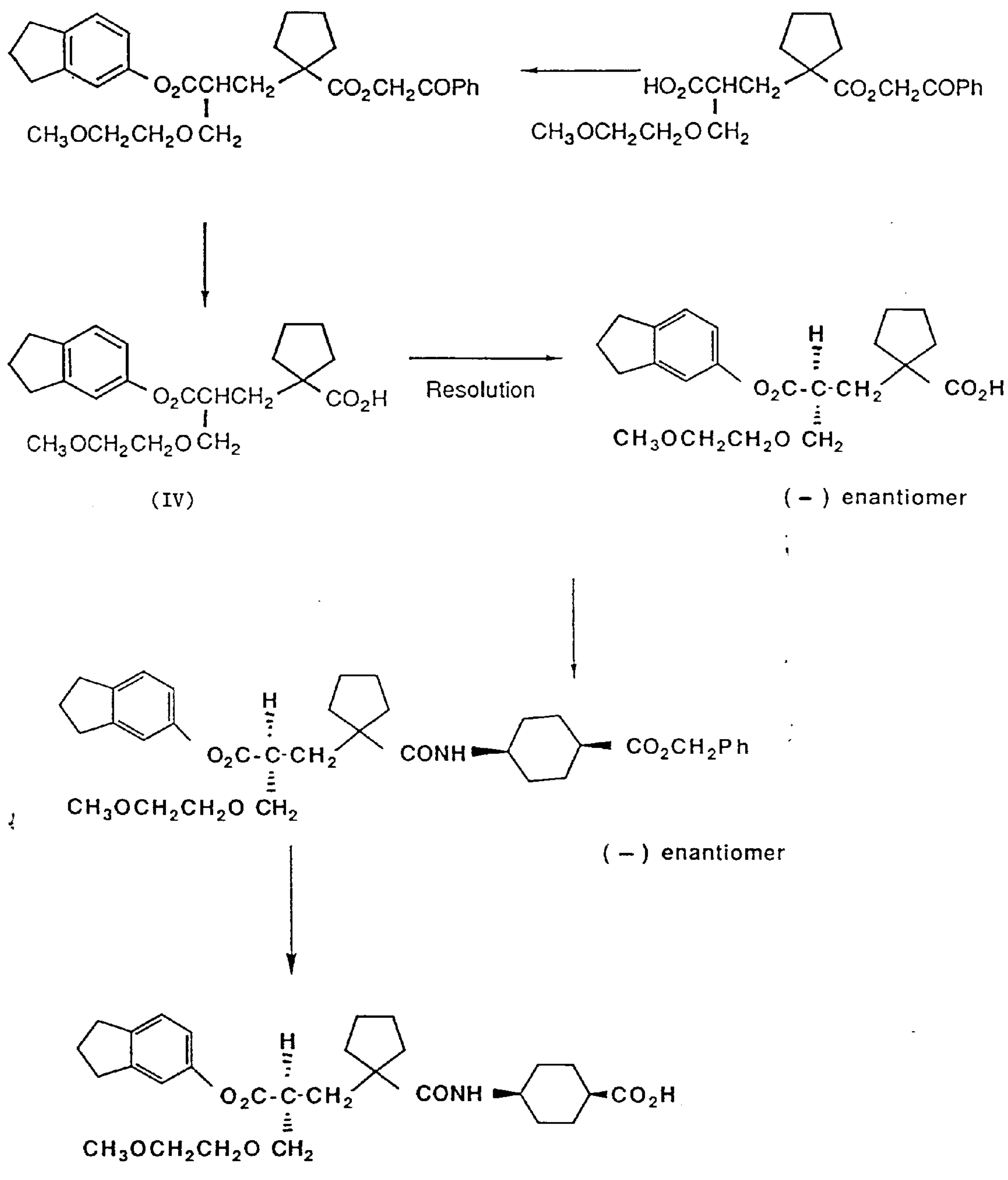


(IV)

(−) enantiomer

(−) enantiomer

(V) (S) (-) enantiomer

In this process, a similar sequence is followed but the indanyl ester (IV) is resolved by fractional crystallisation of its (+) pseudoephedrine salt. A solution of the separated salt is acidified and the free carboxylic acid isolated as the pure S(-) enantiomer. Other salts which can be used as resolving agents for this step include for example, salts with 1-cinchonidine, 1-ephedrine, S(-)alpha-methylbenzylamine, (S,S) (+)2-amino-1-phenyl-1,3-propanediol, L-phenylalaninol and dehydroabietylamine. The absolute stereochemistry was established to be S by comparison with material prepared by asymmetric synthesis. Optical purity was established by chiral NMR assay. This product is coupled with benzyl cis-4-amino-1-cyclohexanecarboxylate as previously described and the benzyl group subsequently removed by catalytic hydrogenation to yield the laevotrotatory S enantiomeric indanyl ester (V).

Enzymatic hydrolysis of this product was shown to give the dextrorotatory S enantiomer of the diacid (III).

Another process for obtaining either the dicarboxylic acid of formula (III) or its indanyl ester of formula (V) is shown in Scheme 3. In this process 1-[2-tert-butoxycarbonyl)-3-(2-methoxyethoxy)propyl]-1-cyclopentanecarboxylic acid is resolved by fractional crystallisation of its (+) pseusoephedrine salt. The alternative salts identified above identified above may also be employed in this step. The (+) enantiomer is then coupled to benzyl cis-4-aminocyclohexane carboxylate using, for example, propanephophonic acid cyclic anhydride as the condensing agent. The t-butyl ester group is removed by treatment with trifluoroacetic acid to yield the mono benzyl ester. This may

Scheme 3

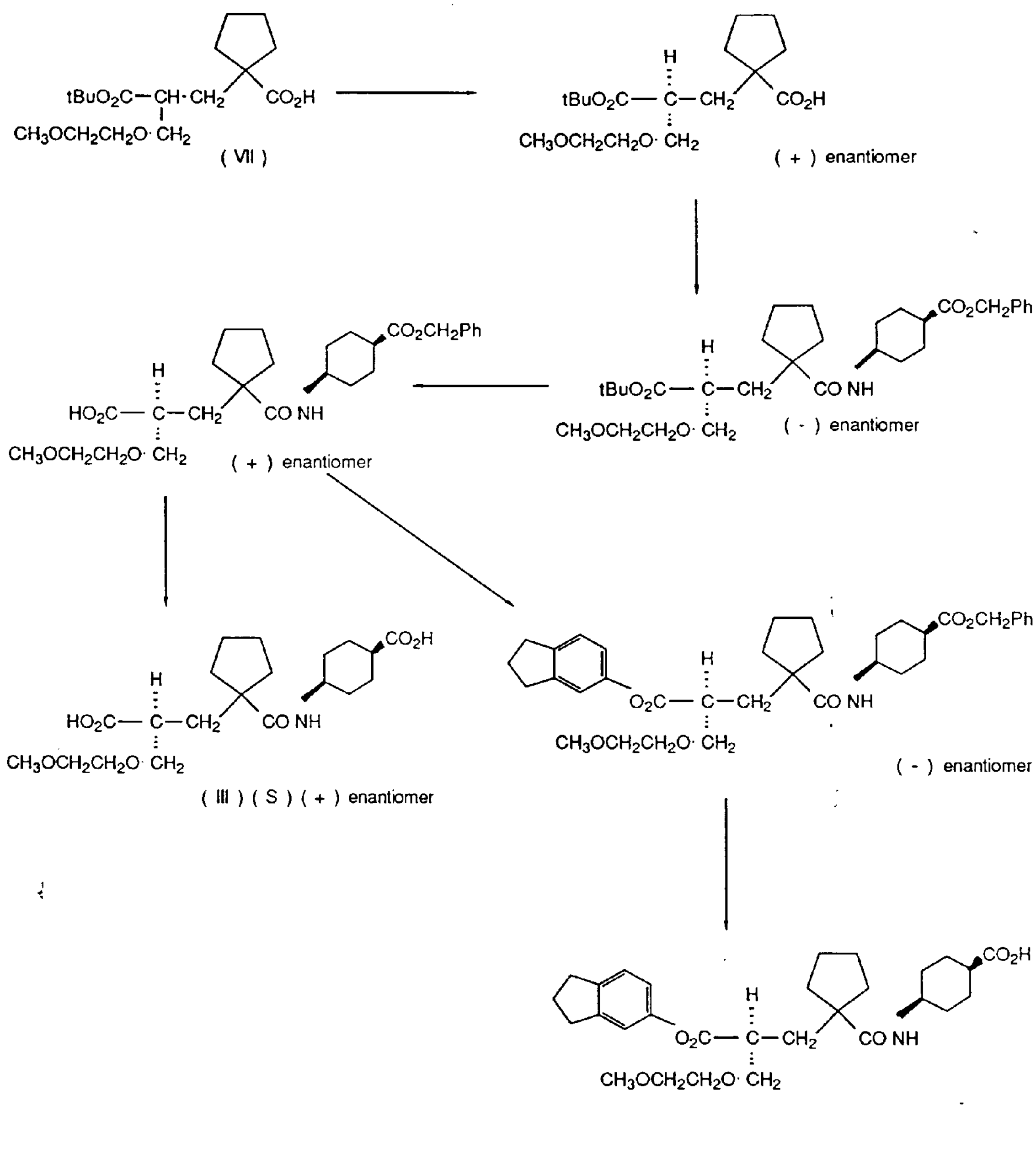

( VII )

( + ) enantiomer

( - ) enantiomer

( + ) enantiomer

( III ) ( S ) ( + ) enantiomer

( - ) enantiomer

( V ) ( S ) ( - ) enantiomer

then either be subjected to catalytic hydrogenation to yield the dicarboxylic acid (III), or esterified with 5-indanol followed by catalytic hydrogenation to yield the indanyl ester (V).

Appropriate reagents and conditions for the various coupling and deprotection steps, described above, together with procedures for determining the biological activity of the products of formula (II) and appropriate pharmaceutical compositions and dosage ranges for their use are described in European patent application

no. 0274234.

The invention will now be more particularly illustrated by reference to the following experimental examples. The purity of compounds was routinely monitored by thin layer chromatography using Merck Kieselgel 60 $F_{254}$ plates. $^1$H-Nuclear magnetic resonance spectra were recorded using a Nicolet QE-300 spectrometer and were in all cases consistent with the proposed structures.

EXAMPLE 1

(2S)-(2-Methoxyethoxymethyl)-3-[1-(phenacyloxycarbonyl)cyclopentyl]propanoic acid N-acetyl-(1R,2S)-ephedrine ester

N,N'-Dicyclohexylcarbodiimide (5.66 g, 24.5 mmole) was added to an ice cold, stirred solution of N-acetyl-(1R,2S)-ephedrine (4.24 g, 20.46 mmole), 2-(2-methoxyethoxymethyl)-3-[1-(phenacyloxycarbonyl)cyclopentyl]propanoic acid (8.43 g, 21.48 mmole) and 4-dimethylaminopyridine (1.23 g, 10 mmole) in dry methylene chloride (100 ml). After one hour the solution was allowed to warm to ambient temperature and stirred for 2½ days. The suspension was filtered, the solvent evaporated under reduced pressure and the residue partitioned between diethyl ether and water. The organic layer was washed sequentially with 0.5 N hydrochloric acid, water, saturated aqueous sodium bicarbonate, and water. Drying ($MgSO_4$) and evaporation gave the crude mixture of diastereoisomers as an oil (12.5 g), which was chromatographed on silica eluting with hexane containing increasing proportions of ethyl acetate (4:6 to 1:9). The faster running component, having Rf 0.45 (silica; ethyl acetate) was the desired diastereoisomer and was obtained following evaporation of the relevant fractions as a gum (5.21 g, 44%). $[\alpha]_D^{25}$ -34.1°, $[\alpha]_{365}^{25}$ - 111.0° (c = 1.0, $CH_2Cl_2$). Found: C,68,19; H,7.59; N,2.46. $C_{33}H_{43}NO_8$ requires C,68.14; H,7.45, N,2.41%

The other diastereoisomer had an Rf of 0.35 (silica; ethyl acetate); $[\alpha]_D^{25}$ - 21.5°, $[\alpha]_{365}^{25}$ -67.3° (c = 1.0, $CH_2Cl_2$).

EXAMPLE 2

(2S)-(2-Methoxyethoxymethyl)-3-(1-carboxycyclopentyl)propanoic acid N-acetyl-(1R,2S)-ephedrine ester

A solution of (2S)-(2-methoxyethoxymethyl)-3-[1-(phenacyloxycarbonyl)cyclopentyl]propanoic acid N-acetyl-(1R,2S)-ephedrine ester (5.17 g, 8.89 mmole) in glacial acetic acid (40 ml) was stirred with activated zinc dust (3.0 g, 47.7 mmole) at room temperature under nitrogen for two hours. The mixture was filtered and the filtrate evaporated to dryness under vacuum, traces of acetic acid being removed by azeotroping with toluene. The residue was dissolved in diethyl ether and the solution extracted with 1N sodium hydroxide solution (12 ml) and washed with water. The combined extracts were acidified with concentrated hydrochloric acid and extracted with diethyl ether. The ether extracts were washed with saturated brine, dried ($MgSO_4$) and evaporated to give the title product as a thick oil (4.03 g, 98%). Found: C,63.96; H,8.21; N,2.87. $C_{25}H_{37}NO_7$ (0.3 $H_2O$) requires C,64.03; H,8.08; N,2.99%. $[\alpha]_D^{25}$ -34.9°, $[\alpha]_{365}^{25}$ -115.4° (c = 1.03, $CH_2Cl_2$).

EXAMPLE 3

3-{1-[(cis-4-Ethoxycarbonylcyclohexyl)carbamoyl]cyclopentyl}-(2S)-(2-methoxyethoxymethyl)propanoic acid N-acetyl-(1R,2S)-ephedrine ester

1-Ethyl-3-(3-dimethylaminopropyl)carbodiimide hydrochloride (3.32 g, 17.34 mmole) was added to an ice cold stirred mixture of the product of Example 2 (3.98 g, 8.58 mmole), cis-4-aminocyclohexanecarboxylic acid ethyl ester hydrochloride (2.70 g, 13 mmole), 1-hydroxybenzotriazole (1.17 g, 8.67 mmole) and N-methylmorpholine (3.07 g, 30.34 mmole) in dry methylene chloride (30 ml). After 15 minutes the mixture was allowed to warm to ambient temperature and to stand overnight. The solvent was evaporated under vacuum and the residue partitioned between diethyl ether and water. The organic layer was washed sequentially with water, 2N-hydrochloric acid, water, saturated aqueous sodium bicarbonate and water. The solution was dried ($MgSO_4$) and the solvent evaporated to give a gum which was chromatographed on silica eluting with ethyl acetate. Further chromatography of the product containing fractions on silica eluting with a mixture of hexane and ethyl acetate (15:85) gave the title compound as a gum (4.65 g, 88%). $[\alpha]_D^{25}$ -30.3°, $[\alpha]_{365}^{25}$ -101.3° (c = 1.01, $CH_2Cl_2$). Found: C,66.16; H,8.66; N,4.45. $C_{34}H_{52}N_2O_8$ requires C,66.21; H,8.50; N,4.54%.

EXAMPLE 4

(S)-cis-4-{1-[2-Carboxy-3-(2-methoxyethoxy)propyl]-1-cyclopentanecarboxamido}-1-cyclohexanecarboxylic acid

The diester product from Example 3 (4.52 g, 7.33 mmole) in a mixture of ethanol (50 ml) and water (50 ml) was hydrogenated over 10% palladium on charcoal catalyst (2.5 g) at 60 p.s.i. (4.1 bar) at room temperature for 24 hours. The mixture was filtered and the filtrate evaporated under reduced pressure. The residue was taken up in diethyl ether and the mono-ester product was extracted into 1N sodium hydroxide (30 ml) the ether being washed with water (30 ml). The combined aqueous extracts were washed with diethyl ether and allowed to stand at room temperature for three days. The solution was saturated with salt, acidified with concentrated hydrochloric acid and extracted with methylene chloride. The organic extract was washed with saturated brine, dried ($MgSO_4$) and the solvent evaporated. Recrystallisation from a mixture of hexane and ethyl acetate gave the title product as a white solid (2.32 g, 79%), m.p. 107.5-108°C. $[\alpha]_D^{25}$ +2.7°, $[\alpha]_{365}^{26}$ +5.1° (c = 1.58, $CH_2Cl_2$). Found: C,60.18; H,8.44; N,3.82. $C_{20}H_{33}NO_7$ requires C,60.13; H,8.33; N,3.51%.

EXAMPLE 5

Phenacyl 1-[2-(5-indanyloxycarbonyl)-3-(2-methoxyethoxy)propyl]-1-cyclopentanecarboxylate

1-(3-Dimethylaminopropyl)-3-ethylcarbodiimide hydrochloride (31.1 g, 0.1625 mole) was added to a stirred solution of 2-(2-methoxyethoxymethyl)-3-[1-(phenacyloxycarbonyl)cyclopentyl]propanoic acid (49 g, 0.125 mole), 5-indanol (83.6 g, 0.625 mole), 1-hydroxybenzotriazole hydrate (18.6 g, 0.1375 mole) and N-methylmorpholine (16.3 g, 0.1625 mole), in methylene chloride (100 ml). The solution was stirred at ambient temperature for 18 hours, diluted with further methylene chloride (300 ml) and washed sequentially with water (2 x 100 ml), 2N hydrochloric acid (2 x 100 ml) and saturated aqueous sodium bicarbonate (2 x 100 ml). Drying ($MgSO_4$) and evaporation gave an oil (129 g) which was chromatographed on silica (1 kg) eluting with hexane containing increasing proportions of ethyl acetate (4:1 to 2:1) to give the title diester as a pale yellow oil (54.5 g; 86%), Rf. 0.54 (silica; hexane, ethyl acetate (2:1).

EXAMPLE 6

1-[2-(5-Indanyloxycarbonyl)-3-(2-methoxyethoxy)propyl]-1-cyclopentanecarboxylic acid

Activated zinc dust (36 g, 0.554 mole) was added portionwise over 45 minutes to a stirred solution of the diester from Example 5 (54 g, 0.106 mole) in glacial acetic acid (378 ml), the temperature being allowed to rise to 32°C. After stirring for 18 hours a further portion of activated zinc dust (36 g, 0.554 mole) was added and the mixture stirred for another hour. The reaction mixture was filtered and the filtrate was evaporated to an oil (46 g) which was chromatographed on silica (500 g) eluting with hexane containing increasing proportions of ethyl acetate (4:1 to 1:1) to give the title ester as a colourless oil (37.8 g, 91.5%) Rf. 0.23 (silica; hexane, ethyl acetate 2:1).

This product could be further characterized as its isopropylamine salt m.p. 76-8°C (hexane). Found: C,66.19; H,8.64; N,3.04. $C_{23}H_{39}NO_6$ requires C,66.79; H,8.75; N,3.12%.

EXAMPLE 7

(S)-1-[2-(5-Indanyloxycarbonyl)-3-(2-methoxyethoxy)propyl]-1-cyclopentanecarboxylic acid

A hot solution of (+)pseudoephedrine (1.98 g) in ethyl acetate (6 ml) was run into a cooled and stirred solution of 1-[2-(5-indanyloxycarbonyl)-3-(2-methoxyethoxy)propyl]-1-cyclopentanecarboxylic acid (4.68 g) in toluene (6 ml), the temperature being allowed to rise to 35°C. The resulting clear solution was chilled to induce crystallisation and granulated at 5°C for several hours. Filtration and drying gave the crude (+)pseudoephedrine salt of the (S)-acid (4.0 g, 60%) as a white solid m.p. 98-102°C. Recrystallisation of 3.5 g of this material from a mixture of toluene (10.5 ml) and ethyl acetate (10.5 ml) gave the (+)-pseudoephedrine salt of the title compound (2.2 g, 62.8% recovery) as white crystals m.p. 111-3°C, $[\alpha]_D$ + 25.1° (c=5, MeOH). Found: C,69.19; H,8.20; N,2.38. $C_{32}H_{45}NO_7$ requires C,69.16; H,8.16; N,2.51%.

A sample of this salt (2 g) was suspended in a mixture of hexane (5 ml), ethyl acetate (5 ml) and water (10

ml) and concentrated hydrochloric acid was added dropwise to adjust the pH of the aqueous phase to 1.5. The two phases of the solution were separated, and the aqueous phase was washed with a 1:1 ethyl acetate-hexane mixture (10 ml). Evaporation of the combined organic layers gave the title compound as a colourless oil (1.2 g, 85% from salt), $[\alpha]_D$ - 3.5° (c=5, MeOH), Rf. 0.41 (silica; toluene, acetic acid 8:2). Found: C,67.25; H,7.77. $C_{22}H_{30}O_6$ requires C,67.67; H,7.74%. A chiral NMR assay of this product showed it to be substantially pure S enantiomer containing only 4% of the R enantiomer.

EXAMPLE 8

(S)-Benzyl cis-4-{1-[2-(5-indanyloxycarbonyl)-3-(2-methoxyethoxy)propyl]-1-cyclopentanecarboxamido}-1-cyclohexanecarboxylate

1-(3-Dimethylaminopropyl)-3-ethylcarbodiimide hydrochloride (337.5 mg, 1.76 mmole) was added to a stirred solution of (S)-1-[2-(5-indanyloxycarbonyl)-3-(2-methoxyethoxy)propyl]-1-cyclopentanecarboxylic acid (625 mg, 1.6 mmole), benzyl cis-4-amino-1-cyclohexanecarboxylate p-toluenesulphonate. (700 mg, 1.73 mmole), 1-hydroxybenzotriazole hydrate (240 mg, 1.78 mmole) and N-methylmorpholine (560 mg, 5.5 mmole) in methylene chloride (3.75 ml). The solution was stirred at ambient temperature for eighteen hours, evaporated under vacuum and the residue partitioned between diethyl ether and water. The organic extract was washed sequentially with 1N hydrochloric acid, saturated aqueous sodium bicarbonate, and water. Drying ($MgSO_4$) and evaporation gave an oil (0.9 g) which was chromatographed on silica (25 g) eluting with hexane containing increasing proportions of ethyl acetate (4:1 to 3:1) to give the required diester as an oil (830 mg, 86%) $[\alpha]_D$ - 3.3° (c=1, MeOH), Rf. 0.52 (silica: ethyl acetate). Found: C,70.32; H,7.74; N,2.19. $C_{36}H_{47}NO_7(0.5\ H_2O)$ requires C,70.33; H,7.87; N,2.28%.

EXAMPLE 9

(S)-cis-4-{1-[2-(5-Indanyloxycarbonyl)-3-(2-methoxyethoxy)propyl]-1-cyclopentanecarboxamido}-1-cyclohexanecarboxylic acid

A solution of (S)-benzyl cis-4-{1-[2-(5-indanyloxycarbonyl)-3-(2-methoxyethoxy)propyl]-1-cyclopentanecarboxamido}- 1-cyclohexanecarboxylate (597 mg, 0.986 mmole) in 5% aqueous ethanol (10 ml) was hydrogenated over 10% palladium on charcoal catalyst (60 mg) at 60 p.s.i. (4.1 bar) and room temperature for 3.5 hours. The catalyst was removed by filtration and the filtrate evaporated under vacuum. The residue was dissolved in diethyl ether (50 ml) and the solution was clarified by filtration, and concentrated to low volume (about 5 ml) when crystallisation occurred. After granulation, filtration and drying gave the title ester (390 mg, 77%) as white crystals m.p. 107-9°C, $[\alpha]_D$ - 5.8° (c=1, MeOH), Rf. 0.40 (silica; toluene, dioxan, acetic acid 90:24:5). Found: C,67.45; H,8.18; N,2.63. $C_{29}H_{41}NO_7$ requires C,67.55; H,8.01; N,2.72%.

EXAMPLE 10

(S)-1-[2-(tert-Butoxycarbonyl)-3-(2-methoxyethoxy)propyl]-1-cyclopentanecarboxylic acid.

A solution of 1-[2-(tert-butoxycarbonyl)-3-(2-methoxyethoxy) propyl]-1-cyclopentanecarboxylic acid (110.1 g, 0.333 mole) in hexane (550 ml) was treated with (+) pseudoephedrine (55.1 g, 0.333 mole) and the mixture was heated to reflux. The resulting solution was cooled to induce crystallisation, and stirred at 5°C for 1 hour to granulate the crystals. After overnight refrigeration at 5°C, filtration, washing with hexane (200 ml) and drying gave the crude (+) pseudoephedrine salt of the (S) acid (89.9 g, 54.4%) as a white solid m.p. 76°-80°C. Recrystallisation of 30 g of this material twice from hexane (225 ml) gave the (+)pseudoephedrine salt of the title compound (21.45 g, 71.5% recovery) as white crystals m.p. 86-7°C, $[\alpha]_D$ + 34.9° (c=1, MeOH). Found: C,65.21; H,9.23; N,2.91. $C_{27}H_{45}NO_7$ requires C,65.42; H,9.15; N,2.82%.

A sample of this salt (10 g) was suspended in hexane (50 ml), and treated with 2N hydrochloric acid (15 ml) (the pH of the aqueous phase was 1.5). The two phases of the solution were separated, and the hexane phase was washed with water (15 ml). Evaporation of the organic layer gave the title compound as a colourless oil (6.3 g, 94% from salt), $[\alpha]_D$ + 2.9° (c=2, MeOH), Rf. 0.44 (silica; diethyl ether, hexane, acetic acid (75:25:1) Found: C,61.41; H,9.17. $C_{17}H_{30}O_6$ requires C,61.79; H,9.15%. A chiral NMR assay of this product showed it to be substantially pure (S) enantiomer containing only 3% of the (R) enantiomer.

EXAMPLE 11

(S)-Benzyl cis-4-{1-[2-(tert-butoxycarbonyl)-3-(2-methoxyethoxy) propyl]-1-cyclopentanecarboxamido}-1-cyclohexanecarboxylate.

A solution of (S)-1-[2-(tert-butoxycarbonyl)-3-(2-methoxyethoxy) propyl]-1-cyclopentanecarboxylic acid (6.61 g, 0.02 mole) in methylene chloride (40 ml) was treated with benzyl cis-4-amino-1-cyclohexane carboxylate p-toluenesulphonate (8.11g, 0.02 mole) and water (26 ml) adjusted to pH 9.5 with 5N aqueous sodium hydroxide. To the stirred two phase solution was added propanephosphonic acid cyclic anhydride (17.8 g of commerical 50% $^{w}/w$ solution in methylene chloride, 0.028 mole) over 45 minutes with dropwise addition of 5N aqueous sodium hydroxide solution to maintain the pH at 8.5. The mixture was stirred for 18 hours and treated with further benzyl cis-4-amino-1-cyclohexanecarboxylate p-toluenesulphonate (2.03 g, 0.005 mole) and propanephosphonic acid cyclic anhydride (12.7 g 50% $^{w}/w$ solution, 0.02 mole), maintaining the pH of the aqueous phase at 8.5 by addition of 5N aqueous sodium hydroxide solution. After stirring for another hour the phases were separated and the organic phase was washed with water (20 ml) and evaporated to an oil (13.04 g) which was chromatographed on silica (300 g). Elution with hexane containing increasing proportions of ethyl acetate (4:1 to 7:3) gave the required diester as an oil (8.12 g, 79.1%) $[\alpha]_D$ - 0.4° (c=2, MeOH), Rf. 0.55 (silica: ethyl acetate).

EXAMPLE 12

(S)-Benzyl cis-4-{1-[2-carboxy-3-(2-methoxyethoxy)propyl]-1-cyclopentanecarboxamido}-1-cyclohexanecarboxylate.

To (S)-benzyl cis-4-{1-[2-tert-butoxycarbonyl)-3-(2-methoxyethoxy) propyl]-1-cyclopentanecarboxamido}-1-cyclohexanecarboxylate (50 g, 0.0917 mole) was added trifluoroacetic acid (100 ml; 1.298 mole) with stirring and cooling to maintain the temperature below 25°C. The solution was allowed to stand for 18 hours, evaporated under vacuum, and the residue (50.2 g) dissolved in ethyl acetate (250 ml). The solution was washed with water (250 ml) adjusted to pH 3.0 with a little saturated aqueous sodium carbonate solution, and then with further water (30 ml). The organic layer was evaporated to give the title compound as a pale amber oil (44.19 g, 98.4%), $[\alpha]_D$ + 0.9°, (c=1, MeOH), Rf. 0.76 (silica;methylene chloride, methanol, acetic acid 90:10:1).

EXAMPLE 13

(S)-Benzyl cis-4-{1-[2-(5-indanyloxycarbonyl)-3-(2-methoxyethoxy) propyl]-1-cyclopentanecarboxamido}-1-cyclohexanecarboxylate.

A solution of (S)-benzyl cis-4-{1-[2-carboxy-3-(2-methoxyethoxy) propyl]-1-cyclopentanecarboxamido}-1-cyclohexanecarboxylate (12.2 g, 0.025 mole) in methylene chloride (12.2 ml) was treated with 5-indanol (6.7 g, 0.05 mole) and then with 1-propanephosphonic acid cyclic anhydride (52.3 g of commerical 50% $^{w}/w$ solution in methylene chloride, 0.0825 mole). The solution was stirred for 17 hours at ambient temperature, and washed sequentially with water (50 ml), 0.5M aqueous potassium hydroxide (20 ml) and water (12 ml). Drying ($MgSO_4$) and evaporation gave an oil (16.54 g) which was chromatographed on silica (60 g) eluting with hexane containing increasing proportions of ethyl acetate (3:1 to 1:1) to give the title diester as a pale yellow oil (10.9 g; 72.1%), $[\alpha]_D$ - 3.3° (c=1, MeOH), Rf. 0.52 (silica; ethylacetate), $R_f$ 0.35 (silica; ethyl acetate, toluene 1:1). This material is identical to that described in Example 8, and is converted in identical manner to (S)-cis-4-{1-[2-(5-indanyloxycarbonyl)-3-(2-methoxyethoxy)propyl]-1-cyclopentanecarboxamido}- 1-cyclohexanecarboxylic acid (as described in Example 9).

EXAMPLE 14

(S)-cis-4-{1-[2-Carboxy-3-(2-methoxyethoxy)propyl]-1-cyclopentanecarboxamido}-1-cyclohexanecarboxylic acid.

A solution of (S)-benzyl cis-4-{1-[2-carboxy-3-(2-methoxyethoxy) propyl]-1-cyclopentanecarboxamido}-1-cyclohexanecarboxylate (4.0 g, 8.18 mmole) in 5% aqueous ethanol (20 ml) was hydrogenated over 5% palladium on charcoal catalyst (0.4 g 50% wet catalyst) at 60 p.s.i. (4.1 bar) and room temperature for 18 hours. The catalyst was removed by filtration and the filtrate was evaporated under vacuum. The residue (3.42 g) was

recrystallised from ethyl acetate (13.7 ml) to give the title diacid (2.15 g, 63%) as white crystals m.p. 108.5°-9.1°C, $[\alpha]_D$ + 1.4° (c=1, MeOH), Rf. 0.55 (silica; methylene chloride, methanol, acetic acid 90:10:1). Found C,60.11; H,8.34; N,3.36. $C_{20}H_{33}NO_7$ requires C,60.13; H,8.33; N,3.51%.
This material is identical to that described in Example 4. A chiral NMR assay of this product showed it substantially pure (S) enantiomer containing only 3% of the (R) enantiomer.

ACTIVITY DATA

The activity of the racemate and separated enantiomers of the cis-4-{1-[2-carboxy-3-(2-methoxyethoxy)propyl]-1-cyclopentanecarboxamido}-1-cyclohexanecarboxylic acid was assessed by measuring their ability to inhibit the neutral endopeptidase E.C.3.4.24.11 in vitro or to induce natriuresis in mice in vivo following the procedure described in European patent application 0274234.

| Enantiomer | $IC_{50}$ against E.C.3.4.24.11 (molar) | Natriuresis in mouse (i.v.) |
|---|---|---|
| (±) R,S | $4.8 \times 10^{-8}$ | active at 3 mg/kg |
| (±) S | $3.9 \times 10^{-8}$ | active at 1.5 mg/kg |
| (-) R | less than $10^{-6}$ | inactive at 3 mg/kg |

**Claims**

**Claims for the following Contracting States: BE, CH, DE, FR, IT, LI, LU, NL, SE, AT**

1. An S enantiomeric compound of the formula:

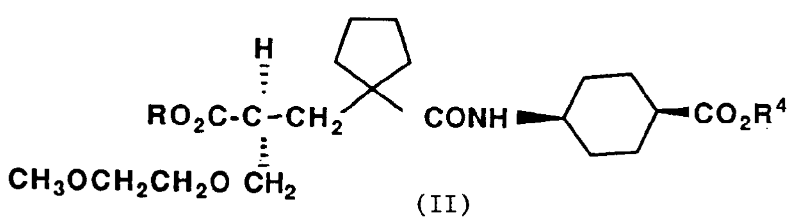

(II)

and pharmaceutically acceptable salts thereof, wherein each R and $R^4$ is H, or one of R and $R^4$ is H and the other is a biolabile ester group, said enantiomer being substantially free of the R-enantiomer.

2. A compound of the formula (II) wherein said biolabile ester group is:-
ethyl
benzyl
1-(2,2-diethylbutyryloxy)ethyl
2-ethylpropionyloxymethyl
1-(2-ethylpropionyloxy)ethyl
1-(2,4-dimethylbenzoyloxy)ethyl
α-benzoyloxybenzyl

1-(benzoyloxy)ethyl
2-methyl-1-propionyloxy-1-propyl
2,4,6-trimethylbenzoyloxymethyl
1-(2,4,6-trimethylbenzoyloxy)ethyl
pivaloyloxymethyl
phenethyl
pphenpropyl
2,2,2-trifluoroethyl
1- or 2-naphthyl
2,4-dimethylphenyl
4-t-butylphenyl
and 5-indanyl.

3. (S)-cis-4-{1-[2-(5-Indanyloxycarbonyl)-3-(2-methoxyethoxy)propyl]-1-cyclopentanecarboxamido}- 1-cyclohexanecarboxylic acid.

4. (-)-cis-4-{1-[2-(5-Indanyloxycarbonyl)-3-(2-methoxyethoxy)propyl]-1-cyclopentanecarboxamido}- 1-cyclohexanecarboxylic acid.

5. (S)-cis-4-{1-[2-Carboxy-3-(2-methoxyethoxy)propyl]-1-cyclopentanecarboxamido}-1-cyclohexanecarboxylic acid.

6. (+)-cis-4-{1-[2-Carboxy-3-(2-methoxyethoxy)propyl]-1-cyclopentanecarboxamido}-1-cyclohexanecarboxylic acid.

7. A process for preparing a compound as claimed in claim 1 which comprises the steps of

(a) coupling an S enantiomeric compound of the formula:-

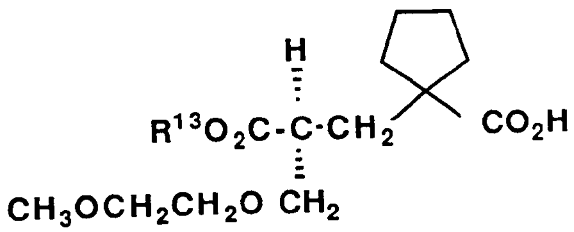

---(VI)

with a compound of the formula:

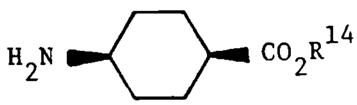

wherein $R^{13}$ and $R^{14}$ are as defined for R and $R^4$ other than H, or are selectively removable carboxylic acid protecting groups, and

(b) removing one or both of $R^{13}$ and $R^{14}$ to yield the mono-ester or dicarboxylic acid product of formula (II); or

(c) removing $R^{13}$, esterifying the product to provide a biolabile ester group and removing $R^{14}$ to yield the product of formula (II) wherein $R^4$ is H and R is a biolabile ester group.

8. A process as claimed in claim 7 wherein $R^{13}$ forms an N-acetyl-(1R,2S)-ephedrine ester and $R^{14}$ is ethyl and the ester groups are removed by hydrogenation followed by hydrolysis to yield the compound of formula (II) wherein R and $R^4$ are both H.

9. A process as claimed in claim 7 wherein $R^{13}$ is indanyl and $R^{14}$ is benzyl and said benzyl group is removed to yield the compound of formula (II) wherein R is 5-indanyl and $R^4$ is H.

10. A process as claimed in claim 7 wherein $R^{13}$ is tert-butyl and $R^{14}$ is benzyl and said groups are removed to yield the compound of formula (II) wherein R and $R^4$ are both H.

11. A process as claimed in claim 7 wherein $R^{13}$ is tert-butyl and $R^{14}$ is benzyl and said tert-butyl group is removed, the product esterfied to provide a biolabile ester group at R and the benzyl group is removed.

12. A pharmaceutical composition comprising a compound as claimed in any one of claims 1 to 6, or a pharmaceutically acceptable salt thereof, together with a pharmaceutically acceptable diluent or carrier.

13. A compound as claimed in any one of claims 1 to 6, or a pharmaceutically acceptable salt thereof, for use in medicine, in particular for use as a diuretic agent for the treatment of hypertension and heart failure.

14. A compound of the formula (VI) as defined in claim 7.

15. A compound as claimed in claim 14 wherein $R^{13}$ is 5-indanyl, tert-butyl or forms a N-acetyl-(1R,2S)-ephedrine ester.

**Claims for the following Contracting State: ES**

1. A process for preparing an S enantiomeric compound of the formula:

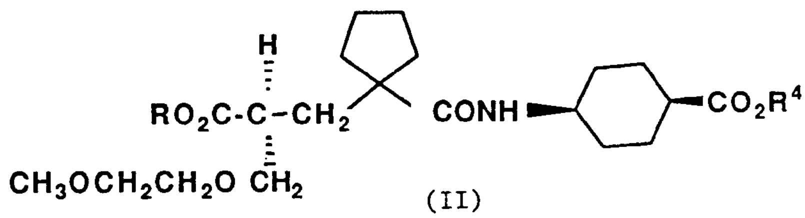

(II)

and pharmaceutically acceptable salts thereof, wherein each R and $R^4$ is H, or one of R and $R^4$ is H and the other is a biolabile ester group, said enantiomer being substantially free of the R-enantiomer, which comprises the steps of

(a) coupling an S enantiomeric compound of the formula:-

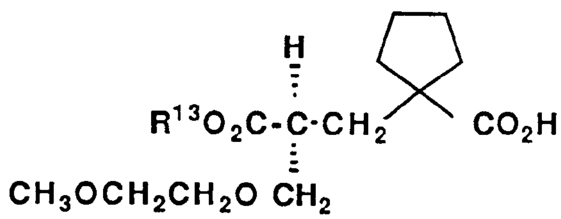

---(VI)

with a compound of the formula:

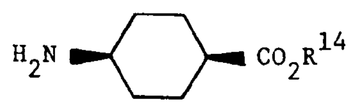

wherein $R^{13}$ and $R^{14}$ are as defined for R and $R^4$ other than H, or are selectively removable carboxylic acid protecting groups, and

(b) removing one or both of $R^{13}$ and $R^{14}$ to yield the mono-ester or dicarboxylic acid product of formula (II), or

(c) removing $R^{13}$, esterifying the product to provide a biolabile ester group and removing $R^{14}$ to yield the product of formula (II) wherein $R^4$ is H and R is a biolabile ester group.

2. A process as claimed in claim 1 wherein $R^{13}$ forms an N-acetyl-(1R,2S)-ephedrine ester and $R^{14}$ is ethyl and the ester groups are removed by hydrogenation followed by hydrolysis to yield the compound of formula (II) wherein R and $R^4$ are both H.

3. A process as claimed in claim 1 wherein $R^{13}$ is idanyl and $R^{14}$ is benzyl and said benzyl group is removed to yield the compound formula (II) wherein R is 5-indanyl and $R^4$ is H.

4. A process as claimed in claim 1 wherein $R^{13}$ is tert-butyl and $R^{14}$ is benzyl and said groups are removed to yield the compound of formula (II) wherein R and $R^4$ are both H.

5. A process as claimed in claim 1 wherein $R^{13}$ is tert-butyl and $R^{14}$ is benzyl and said tert-butyl group is removed, the product esterfied to provide a biolabile ester group at R and the benzyl group is removed.

6. A process as claimed in claim 1 wherein said biolabile ester group is:-

ethyl
benzyl
1-(2,2-diethylbutyryloxy)ethyl
2-ethylpropionyloxymethyl
1-(ethylpropionyloxy)ethyl
1-(2,4-dimethylbenzoyloxy)ethyl
α-benzoyloxybenzyl
1-(benzoyloxy)ethyl
2-methyl-1-propionyloxy-1-propyl
2,4,6-trimethylbenzoyloxymethyl
1-(2,4,6-trimethylbenzoyloxy)ethyl
pivaloyloxymethyl
phenethyl
phenpropyl
2,2,2-trifluoroethyl
1- or 2-naphthyl
2,4-dimethylphenyl
4-t-butylphenyl
or 5-indanyl.

7. A process as claimed in claim 1, claim 2 or claim 4 for producing the compound:
(S)-cis-4-{1-[2-Carboxy-3-(2-methoxyethoxy)propyl]-1-cyclopentanecarboximado}-1-cyclohexanecarboxylic acid.

8. A process as claimed in claim 1, claim 3 or claim 5, for producing the compound:
(S)-cis-4-{1-[2-(5-Indanyloxycarbonyl)-3-(2-methoxyethoxy)propyl]-1-cyclopentanecarboxamido}-1-cyclohexanecarboxylic acid.

**Claims for the following Contracting State : GR**

1. A process for preparing an S enantiomeric compound of the formula:

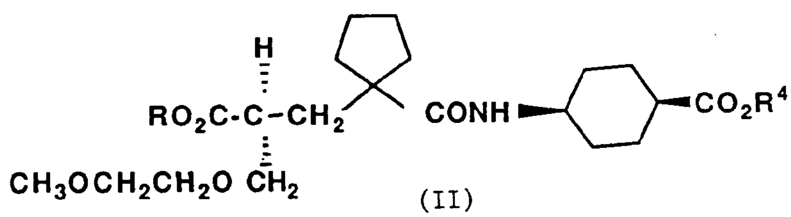

(II)

and pharmaceutically acceptable salts thereof, wherein each of R and $R^4$ is H, or one of R and $R^4$ is H and the other is a biolabile ester group, said enantiomer being substantially free of the R-enantiomer, which comprises the steps of

(a) coupling an S enantiomeric compound of the formula:-

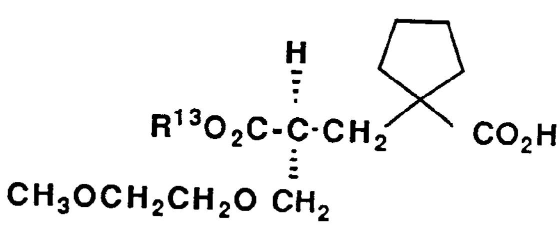

---(VI)

with a compound of the formula:

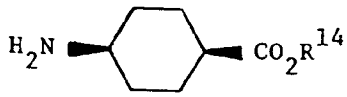

wherein $R^{13}$ and $R^{14}$ are as defined for R and $R^4$ other than H, or are selectively removable carboxylic acid protecting groups, and

(b) removing one or both of $R^{13}$ and $R^{14}$ to yield the mono-ester or dicarboxylic acid product of formula (II). or

(c) removing $R^{13}$, esterifying the product to provide a biolabile ester group and removing $R^{14}$ to yield the product of formula (II) wherein $R^4$ is H and R is a biolabile ester group.

2. A process as claimed in claim 1 wherein $R^{13}$ forms an N-acetyl-(1R,2S)-ephedrine ester and $R^{14}$ is ethyl and the ester groups are removed by hydrogenation followed by hydrolysis to yield the compound of formula (II) wherein R and $R^4$ are both H.

3. A process as claimed in claim 1 wherein $R^{13}$ is idanyl and $R^{14}$ is benzyl and said benzyl group is removed to yield the compound formula (II) wherein R is 5-indanyl and $R^4$ is H.

4. A process as claimed in claim 1 wherein $R^{13}$ is tert-butyl and $R^{14}$ is benzyl and said groups are removed to yield the compound of formula (II) wherein R and $R^4$ are both H.

5. A process as claimed in claim 1 wherein $R^{13}$ is tert-butyl and $R^{14}$ is benzyl and said tert-butyl group is removed, the product esterfied to provide a biolabile ester group at R and the benzyl group is removed.

6. A process as claimed in claim 1 wherein said biolabile ester group is:-
ethyl
benzyl
1-(2,2-diethylbutyryloxy)ethyl
2-ethylpropionyloxymethyl
1-(2-ethylpropionyloxy)ethyl
1-(2,4-dimethylbenzoyloxy)ethyl
α-benzoyloxybenzyl
1-(benzoyloxy)ethyl
2-methyl-1-propionyloxy-1-propyl
2,4,6-trimethylbenzoyloxymethyl
1-(2,4,6-trimethylbenzoyloxy)ethyl
pivaloyloxymethyl
phenethyl
phenpropyl
2,2,2-trifluoroethyl
1- or 2-naphthyl
2,4-dimethylphenyl
4-t-butylphenyl
or 5-indanyl.

7. A process as claimed in claim 1, claim 2 or claim 4 for producing the compound:
(S)-cis-4-{1-[2-Carboxy-3-(2-methoxyethoxy)propyl]-1-cyclopentanecarboximado}-1-cyclohexanecarboxylic acid.

8. A process as claimed in claim 1, claim 3 or claim 5, for producing the compound:
(S)-cis-4-{1-[2-(5-Indanyloxycarbonyl)-3-(2-methoxyethoxy)propyl]-1-cyclopentanecarboxamido}-1-cyclohexanecarboxylic acid.

9. A compound of the formula (VI) as defined in claim 1.

10. A compound as claimed in claim 9 wherein $R^{13}$ is 5-indanyl, tert-butyl or forms a N-acetyl-(1R,22)-ephedrine ester.

## Patentansprüche

### Patentansprüche für folgende Vertragsstaaten : BE, CH, DE, FR, IT, LI, LU, NL, AT, SE

1. S-Enantiomere Verbindung der Formel

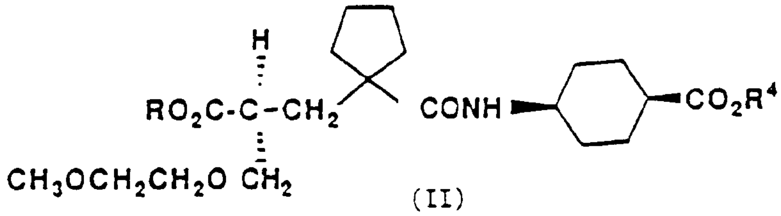

(II)

und pharmazertisch annehmbare Salze derselben, worin R und $R^4$ jeweils H sind oder einer der Substituenten R und $R^4$ H und der andere eine biolabiie Estergruppe ist, wobei dieses Enantiomer im wesentlichen vom R-Enantiomer frei ist.

2. Verbindung der Formel (II), worin die biolabile Estergruppe
Ethyl
Benzyl
1-(2,2-Diethylbutyryloxy)ethyl
2-Ethylpropionyloxymethyl
1-(2-Ethylpropionyloxy)ethyl
1-(2,4-Dimethylbenzoyloxy)ethyl
α-Benzoyloxybenzyl
1-(Benzoyloxy)ethyl
2-Methyl-1-propionyloxy-1-propyl
2,4,6-Trimethylbenzoyloxymethyl
1-(2,4,6-Trimethylbenzoyloxy)ethyl
Pivaloyloxymethyl
Phenethyl
Phenpropyl
2,2,2-Trifluorethyl
1- oder 2-Naphthyl
2,4-Dimethylphenyl
4-t-Butylphenyl oder
5-Indanyl ist.

3. (S)-cis-4-{-1-[2-(5-Indanyloxycarbonyl)-3-(2-methoxyethoxy)propyl]-1-cyclopentancarboxamido}-1-cyclohexancarbonsäure.

4. (-)-cis-4-{-1-[2-(5-Indanyloxycarbonyl)-3-(2-methoxyethoxy)propyl]-1-cyclopentancarboxamido}-1-cyclohexancarbonsäure.

5. (S)-cis-4-{1-[2-Carboxy-3-(2-methoxyethoxy)propyl]-1-cyclopentancarboxamido}-1-cyclohexancarbonsäure.

6. (+)-cis-4-{1-[2-Carboxy-3-(2-methoxyethoxy)propyl]-1-cyclopentancarboxamido}-1-cyclohexancarbonsäure.

7. Verfahren zur Herstellung einer Verbindung gemäß Anspruch 1, das die Stufen

(a) des Kuppelns einer S-enantiomeren Verbindung der Formel

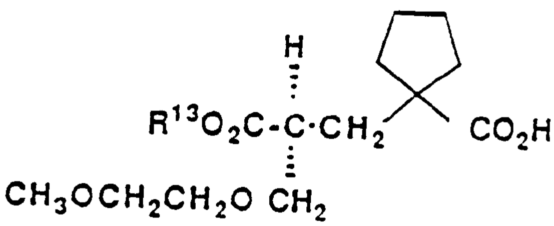

—(VI)

mit einer Verbindung der Formel

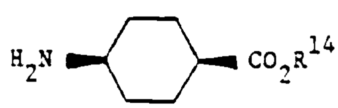

worin $R^{13}$ und $R^{14}$ die gleiche Definition wie R und $R^4$ mit Ausnahme von H haben oder selektiv entfernbare Carbonsäureschutzgruppen sind, und

(b) der Entfernung eines oder beider Substituenten $R^{13}$ und $R^{14}$ zur Bildung des Monoester- oder Dicarbonsäureproduktes der Formel (II), oder

(c) der Entfernung von $R^{13}$, des Verestens des Produktes zur Bildung einer biolabilen Estergruppe und Entfernung von $R^{14}$ zur Bildung des Produktes der Formel (II), worin $R^{14}$ H ist und R eine biolabile Estergruppe ist, umfaßt.

8. Verfahren nach Anspruch 7, worin $R^{13}$ einen N-Acetyl-(1R,2S)-ephedrinester bildet und $R^{14}$ Ethyl ist und die Estergruppen durch Hydrierung entfernt werden, gefolgt von einer Hydrolyse, um die Verbindung der Formel (II) zu ergeben, in welcher R und $R^4$ beide H sind.

9. Verfahren nach Anspruch 7, worin $R^{13}$ Indanyl ist und $R^{14}$ Benzyl ist und die Benzylgruppe entfernt wird, um die Verbindung der Formel (II) zu ergeben, in welcher R 5-Indanyl ist und $R^4$ H ist.

10. Verfahren nach Anspruch 7, worin $R^{13}$ t-Butyl ist und $R^{14}$ Benzvl ist und diese Gruppen entfernt werden, um die Verbindung der Formel (II) zu ergeben, in welcher R und $R^4$ beide H sind.

11. Verfahren nach Anspruch 7, worin $R^{13}$ t-Butyl ist und $R^{14}$ Benzyl ist und die t-Butylgruppe entfernt wird, das Produkt zur Bildung einer biolabilen Estergruppe bei R verestert und die Benzylgruppe entfernt wird.

12. Pharmazeutische Zusammensetzung, die eine Verbindung gemäß irgendeinem der Ansprüche 1 bis 6 oder ein pharmazeutisch annehmbares Salz derselben zusammen mit einem pharmazeutisch annehmbaren Verdünnungsmittel oder Träger umfaßt.

13. Eine Verbindung gemäß irgendeinem der Ansprüche 1 bis 6 oder ein pharmazeutisch annehmbares Salz derselben zur Verwendung in der Medizin, insbesondere zur Verwendung als diuretisches Mittel bei der Behandlung von Hypertension und Herzversagen.

14. Eine Verbindung der Formel (VI) gemäß Anspruch 7.

15. Eine Verbindung gemäß Anspruch 14, in welcher $R^{13}$ 5-Indanyl, t-Butyl ist oder einen N-Acetyl-(1R,2S)-ephedrinester bildet.

**Patentanspruche für folgenden Vertragsstaat : GR**

1. Verfahren zur Herstellung einer S-enantiomeren Verbindung der Formel

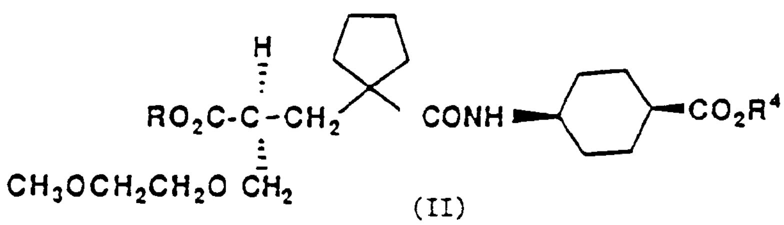

(II)

und pharmazeutisch annehmbarer Salze derselben, worin R und $R^4$ jeweils H sind oder einer der Substituenten R und $R^4$ H und der andere eine biolabile Estergruppe ist, wobei dieses Enantiomer im wesentlichen vom R-Enantiomer frei ist, das die Schritte

(a) des Kuppelns einer S-enantiomeren Verbindung der Formel

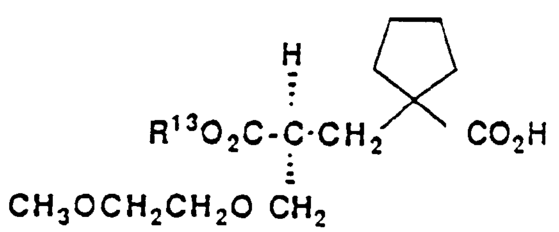

—(VI)

mit einer Verbindung der Formel

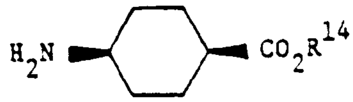

worin $R^{13}$ und $R^{14}$ die gleiche Definition wie R und $R^4$ mit Ausnahme von H haben oder selektiv entfernbare Carbonsäureschutzgruppen sind, und

(b) der Entfernung eines oder beider Substituenten $R^{13}$ und $R^{14}$ zur Bildung des Monoester- oder Dicarbonsäureproduktes der Formel (II), oder

(c) der Entfernung von $R^{13}$, des Veresterns des Produktes zur Bildung einer biolabilen Estergruppe und Entfernung von $R^{14}$ zur Bildung des Produktes der Formel (II), worin $R^4$ H ist und R eine biolabile Estergruppe ist umfaßt.

2. Verfahren nach Anspruch 1, worin $R^{13}$ einen N-Acetyl-(1R,2S)-ephedrinester bildet und $R^{14}$ Ethyl ist und die Estergruppen durch Hydrierung entfernt werden, gefolgt von einer Hydrolyse, um die Verbindung der Formel (II) zu ergeben, in welcher R und $R^4$ beide H sind.

3. Verfahren nach Anspruch 1, worin $R^{13}$ Indanyl ist und $R^{14}$ Benzyl ist und die Benzylgruppe entfernt wird, um die Verbindung der Formel (II) zu ergeben, in welcher R 5-Indanyl ist und $R^4$ H ist.

4. Verfahren nach Anspruch 1, worin $R^{13}$ t-Butyl ist und $R^{14}$ Benzyl ist und diese Gruppen entfernt werden, um die Verbindung der Formel (II) zu ergeben, in welcher R und $R^4$ beide H sind.

5. Verfahren nach Anspruch 1, worin $R^{13}$ t-Butyl ist und $R^{14}$ Benzyl ist und die t-Butylgruppe entfernt wird, das Produkt zur Bildung einer biolabilen Estergruppe bei R verestert und die Benzylgruppe entfernt wird.

6. Verfahren nach Anspruch 1, worin die biolabile Estergruppe
Ethyl
Benzyl
1-(2,2-Diethylbutyryloxy)ethyl
2-Ethylpropionyloxymethyl
1-(2-Ethylpropionyloxy)ethyl
1-(2,4-Dimethylbenzoyloxy)ethyl
α-Benzoyloxybenzyl
1-(Benzoyloxy)ethyl
2-Methyl-1-propionyloxy-1-propyl
2,4,6-Trimethylbenzoyloxymethyl
1-(2,4,6-Trimethylbenzoyloxy)ethyl
Pivaloyloxymethyl
Phenethyl
Phenpropyl
2,2,2-Trifluorethyl
1- oder 2-Naphthyl
2,4-Dimethylphenyl
4-t-Butylphenyl oder
5-Indanyl ist.

7. Verfahren nach Anspruch 1, 2 oder 4 zur Herstellung der Verbindung
(S)-cis-4-{1-[2-Carboxy-3-(2-methoxyethoxy)-propyl]-1-cyclopentancarboxamido}-1-cyclohexancarbonsäure.

8. Verfahren nach Anspruch 1, 3 oder 5 zur Herstellung der Verbindung
(S)-cis-4-{1-[2-(5-Indanyloxycarbonyl)-3-(2-methoxyethoxy)-propyl]-1-cyclopentancarboxamido)-1-cyclohexancarbonsäure.

9. Eine Verbindung der Formel (VI) gemäß Definition in Anspruch 1.

10. Eine Verbindung gemäß Anspruch 9, worin $R^{13}$ 5-Indanyl, t-Butyl ist oder einen N-Acetyl-(1R,2S)-ephedrinester bildet.

**Patentansprüche für folgenden Vertragsstaat :**

1. Verfahren zur Herstellung einer S-enantiomeren Verbindung der Formel

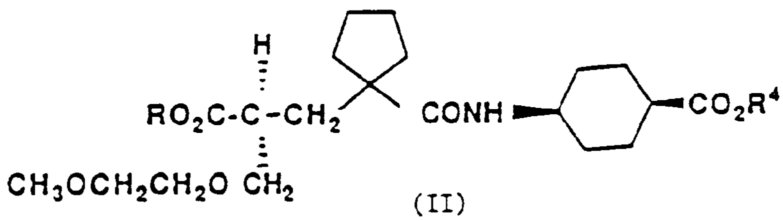

(II)

und pharmazeutisch annehmbarer Salze derselben, worin R und $R^4$ jeweils H sind oder einer der Substituenten R und $R^4$ H und der andere eine biolabile Estergruppe ist, wobei dieses Enantiomer im wesentlichen vom R-Enantiomer frei ist, das die Schritte

(a) des Kuppelns einer S-enantiomeren Verbindung der Formel

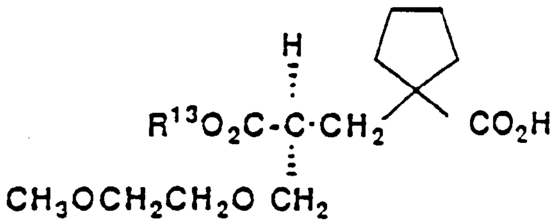

—(VI)

mit einer Verbindung der Formel

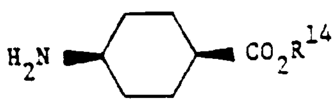

worin $R^{13}$ und $R^{14}$ die gleiche Definition wie R und $R^4$ mit Ausnahme von H haben oder selektiv entfernbare Carbonsäureschutzgruppen sind, und

(b) der Entfernung eines oder beider Substituenten $R^{13}$ und $R^{14}$ zur Bildung des Monoester- oder Dicarbonsäureproduktes der Formel (II), oder

(c) der Entfernung von $R^{13}$, des Veresterns des Produktes zur Bildung einer biolabilen Estergruppe und Entfernung von $R^{14}$ zur Bildung des Produktes der Formel (II), worin $R^4$ H ist und R eine biolabile Estergruppe ist, umfaßt.

2. Verfahren nach Anspruch 1, worin $R^{13}$ einen N-Acetyl-(1R,2S)-ephedrinester bildet und $R^{14}$ Ethyl ist und die Estergruppen durch Hydrierung entfernt werden, gefolgt von einer Hydrolyse, um die Verbindung der Formel (II) zu ergeben, in welcher R und $R^4$ beide H sind.

3. Verfahren nach Anspruch 1, worin $R^{13}$ Indanyl ist und $R^{14}$ Benzyl ist und die Benzylgruppe entfernt wird, um die Verbindung der Formel (II) zu ergeben, in welcher R 5-Indanyl ist und $R^4$ H ist.

4. Verfahren nach Anspruch 1, worin $R^{13}$ t-Butyl ist und $R^{14}$ Benzyl ist und diese Gruppen entfernt werden, um die Verbindung der Formel (II) zu ergeben, in welcher R und $R^4$ beide H sind.

5. Verfahren nach Anspruch 1, worin $R^{13}$ t-Butyl ist und $R^{14}$ Benzyl ist und die t-Butylgruppe entfernt wird, das Produkt zur Bildung einer biolabilen Estergruppe bei R verestert und die Benzylgruppe entfernt wird.

6. Verfahren nach Anspruch 1, worin die biolabile Estergruppe

Ethyl
Benzyl
1-(2,2-Diethylbutyryloxy)ethyl
2-Ethylpropionyloxymethyl
1-(2-Ethylpropionyloxy)ethyl
1-(2,4-Dimethylbenzoyloxy)ethyl
α-Benzoyloxybenzyl
1-(Benzoyloxy)ethyl
2-Methyl-1-propionyloxy-1-propyl
2,4,6-Trimethylbenzoyloxymethyl
1-(2,4,6-Trimethylbenzoyloxy)ethyl
Pivaloyloxymethyl

Phenethyl
Phenpropyl
2,2,2-Trifluorethyl
1- oder 2-Naphthyl
2,4-Dimethylphenyl
4-t-Butylphenyl oder
5-Indanyl ist.

7. Verfahren nach Anspruch 1, 2 oder 4 zur Herstellung der Verbindung
(S)-cis-4-{1-[2-Carboxy-3-(2-methoxyethoxy)-propyl]-1-cyclopentancarboxamido}-1-cyclohexancarbonsäure.

8. Verfahren nach Anspruch 1, 3 oder 5 zur Herstellung der Verbindung
(S)-cis-4-{1-[2-(5-Indanyloxycarbonyl)-3-(2-methoxyethoxy)propyl]-1-cyclopentancarboxamido}-1-cyclohexancarbonsäure.

## Revendications

**Revendications pour les Etats contractants suivants : BE, CH, DE, FR, IT, LI, LU, NL, SE, AT**

1. Composé énantiomère-S de formule :

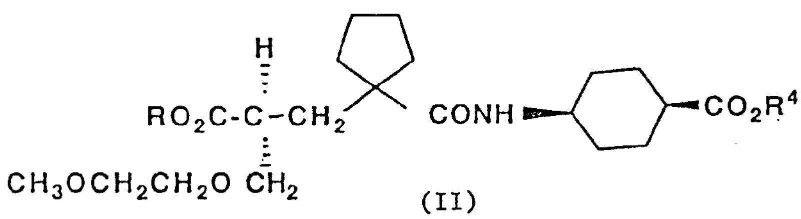

(II)

et sels pharmaceutiquement acceptables de ce composé, dans lequel chacun des radicaux R et $R^4$ représente H, ou l'un des radicaux R et $R^4$ représente H et l'autre est un groupe ester biolabile, ledit énantiomère étant sensiblement dépourvu d'énantiomère-R.

2. Composé de formule (II) dans lequel ledit groupe ester biolabile est un groupe :
éthyle
benzyle
1-(2,2-diéthylbutyryloxy)éthyle
2-éthylpropionyloxyméthyle
1-(2-éthylpropionyloxy)éthyle
1-(2,4-diméthylbenzoyloxy)éthyle
α-benzoyloxybenzyle
1-(benzoyloxy)éthyle
2-méthyl-1-propionyloxy-1-propyle
2,4,6-triméthylbenzoyloxyméthyle
1-(2,4,6-triméthylbenzoyloxy)éthyle
pivaloyloxyméthyle
phénéthyle
phènpropyle
2,2,2-trifluoroéthyle
1- ou 2-naphtyle
2,4-diméthylphényle
4-t-butylphényle
ou 5-indanyle.

3. Acide (S)-cis-4-{1-(2-(5-indanyloxycarbonyl)-3-(2-méthoxyéthoxy)propyl)-1-cyclopentanecarboxamido}-1-cyclohexanecarboxylique.

4. Acide (-)-cis-4-{1-(2-(5-indanyloxycarbonyl)-3-(2-méthoxyéthoxy)propyl)-1-cyclopentanecarboxamido}-1-cyclohexanecarboxylique.

5. Acide (S)-cis-4-{1-(2-carboxy-3-(2-méthoxyéthoxy)propyl)-1-cyclopentanecarboxamido}-1-cyclohexanecarboxylique.

6. Acide (+)-cis-4-{1-(2-carboxy-3-(2-méthoxyéthoxy)propyl)-1-cyclopentanecarboxamido}-1-cyclohexanecarboxylique.

7. Procédé de préparation d'un composé selon la revendication 1, qui consiste

(a) à coupler un composé énantiomère-S de formule :

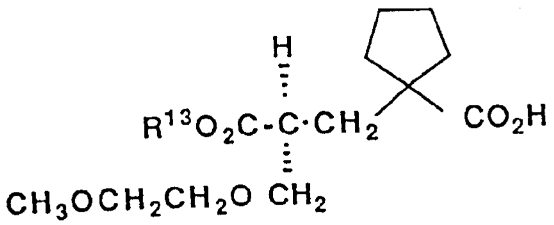

---(VI)

avec un composé de formule :

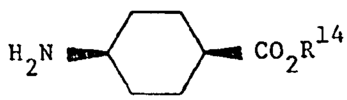

dans laquelle $R^{13}$ et $R^{14}$ sont tels que définis à propos de R et de $R^4$, mais autres que H, ou sont des groupes protecteurs de la fonction acide carboxylique sélectivement éliminables, et

(b) à éliminer l'un des radicaux $R^{13}$ et $R^{14}$, ou les deux, pour donner le produit monoester ou acide dicarboxylique de formule (II) ;

ou (c) à éliminer le radical $R^{13}$, à estérifier le produit pour donner un groupe ester biolabile et à éliminer le radical $R^{14}$ pour donner le produit de formule (II) dans lequel $R^4$ représente H et R est un groupe ester biolabile.

8. Procédé selon la revendication 7, dans lequel $R^{13}$ forme un ester N-acétyl-(1R,2S)-éphédrine et $R^{14}$ est un groupe éthyle, les groupes ester étant éliminés par hydrogénation suivie d'une hydrolyse pour donner le composé de formule (II) dans lequel R et $R^4$ représentent tous deux H.

9. Procédé selon la revendication 7, dans lequel $R^{13}$ est un groupe indanyle et $R^{14}$ est un groupe benzyle, ledit groupe benzyle étant éliminé pour donner le composé de formule (II) dans lequel R est un groupe 5-indanyle et $R^4$ représente H.

10. Procédé selon la revendication 7, dans lequel $R^{13}$ est un groupe tert-butyle et $R^{14}$ un groupe benzyle, lesdits groupes étant éliminés pour donner le composé de formule (II) dans lequel R et $R^4$ représentent tous deux H.

11. Procédé selon la revendication 7, dans lequel $R^{13}$ est un groupe tert-butyle et $R^{14}$ est un groupe benzyle, ledit groupe tert-butyle étant éliminé et le produit estérifié pour donner un groupe ester biolabile au niveau de R et le groupe benzyle est éliminé.

12. Composition pharmaceutique comprenant un composé selon l'une quelconque des revendications 1 à 6, ou un sel pharmaceutiquement acceptable d'un tel composé, avec un diluant ou un véhicule pharmaceutiquement acceptable.

13. Composé selon l'une quelconque des revendications 1 à 6, ou sel pharmaceutiquement acceptable d'un tel composé, pour utilisation en médecine, en particulier pour utilisation comme agent diurétique dans le traitement de l'hypertension et des défaillances cardiaques.

14. Composé de formule (VI) selon la revendication 7.

15. Composé selon la revendication 14, dans lequel $R^{13}$ est un groupe 5-indanyle, tert-butyle ou forme un ester N-acétyl-(1R-2S)-éphédrine.

**Revendications pour l'Etat contractant suivant : ES**

1. Procédé de préparation d'un énantiomère-S de formule :

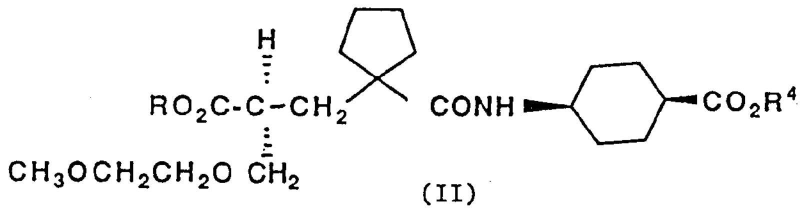

(II)

et de sels pharmaceutiquement acceptables de ce composé, dans lequel chacun des radicaux R et $R^4$ représente H, ou l'un des radicaux R et $R^4$ représente H et l'autre est un groupe ester biolabile, ledit énantiomère étant sensiblement dépourvu d'énantiomère-R, qui consiste

(a) à coupler un composé énantiomère-S de formule :

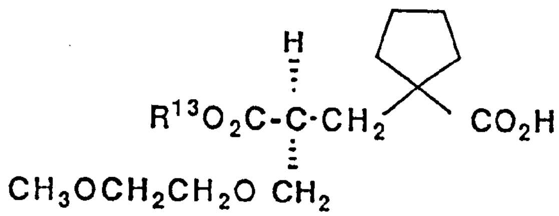

---(VI)

avec un composé de formule :

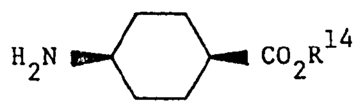

dans laquelle $R^{13}$ et $R^{14}$ sont tels que définis à propos de R et de $R^4$, mais autres que H, ou sont des groupes protecteurs de la fonction acide carboxylique sélectivement éliminables, et

(b) à éliminer l'un des radicaux $R^{13}$ et $R^{14}$, ou les deux, pour donner le produit monoester ou acide dicarboxylique de formule (II) ;

ou (c) à éliminer le radical $R^{13}$, à estérifier le produit pour donner un groupe ester biolabile et à éliminer le radical $R^{14}$ pour donner le produit de formule (II) dans lequel $R^4$ représente H et R est un groupe ester biolabile.

2. Procédé selon la revendication 1, dans lequel $R^{13}$ forme un ester N-acétyl-(1R,2S)-éphédrine et $R^{14}$ est un groupe éthyle, les groupes ester étant éliminés par hydrogénation suivie d'une hydrolyse pour donner le composé de formule (II) dans lequel R et $R^4$ représentent tous deux H.

3. Procédé selon la revendication 1, dans lequel $R^{13}$ est un groupe indanyle et $R^{14}$ est un groupe benzyle, ledit groupe benzyle étant éliminé pour donner le composé de formule (II) dans lequel R est un groupe 5-indanyle et $R^4$ représente H.

4. Procédé selon la revendication 1, dans lequel $R^{13}$ est un groupe tert-butyle et $R^{14}$ un groupe benzyle, lesdits groupes étant éliminés pour donner le composé de formule (II) dans lequel R et $R^4$ représentent tous deux H.

5. Procédé selon la revendication 1, dans lequel $R^{13}$ est un groupe tert-butyle et $R^{14}$ est un groupe benzyle, ledit groupe tert-butyle étant éliminé et le produit estérifié pour donner un groupe ester biolabile au niveau de R et le groupe benzyle est éliminé.

6. Procédé selon la revendication 1, dans lequel ledit groupe ester biolabile est un groupe :
éthyle
benzyle
1-(2,2-diéthylbutyryloxy)éthyle
2-éthylpropionyloxyméthyle
1-(2-éthylpropionyloxy)éthyle
1-(2,4-diméthylbenzoyloxy)éthyle
α-benzoyloxybenzyle

1-(benzoyloxy)éthyle
2-méthyl-1-propionyloxy-1-propyle
2,4,6-triméthylbenzoyloxyméthyle
1-(2,4,6-triméthylbenzoyloxy)éthyle
pivaloyloxyméthyle
phénéthyle
phènpropyle
2,2,2-trifluoroéthyle
1- ou 2-naphtyle
2,4-diméthylphényle
4-t-butylphényle
ou 5-indanyle.

7. Procédé selon la revendication 1, 2 ou 4 de production du composé :

Acide (S)-cis-4-{1-(2-carboxy-3-(2-méthoxyéthoxy)propyl)-1-cyclopentanecarboxamido}-1-cyclohexanecarboxylique.

8. Procédé selon la revendication 1, 3 ou 5 de production du composé :

Acide (S)-cis-4-{1-(2-(5-indanyloxycarbonyl)-3-(2-méthoxyéthoxy)propyl)-1-cyclopentanecarboxamido}-1-cyclohexanecarboxylique.

**Revendication pour l'Etat contractant suivant : GR**

1. Procédé de préparation d'un énantiomère-S de formule :

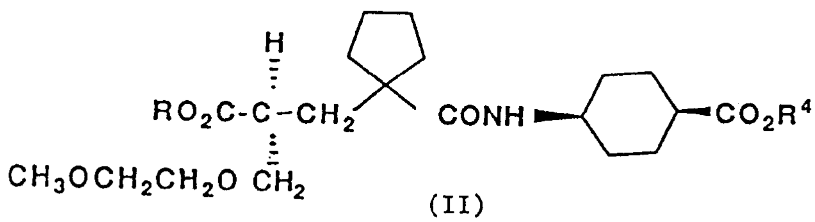

(II)

et de sels pharmaceutiquement acceptables de ce composé, dans lequel chacun des radicaux R et $R^4$ représente H, ou l'un des radicaux R et $R^4$ représente H et l'autre est un groupe ester biolabile, ledit énantiomère étant sensiblement dépourvu d'énantiomère-R, qui consiste

(a) à coupler un composé énantiomère-S de formule :

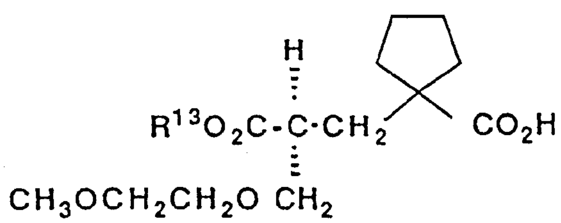

---(VI)

avec un composé de formule :

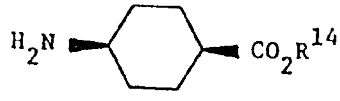

dans laquelle $R^{13}$ et $R^{14}$ sont tels que définis à propos de R et de $R^4$, mais autres que H, ou sont des groupes protecteurs de la fonction acide carboxylique sélectivement éliminables, et

(b) à éliminer l'un des radicaux $R^{13}$ et $R^{14}$, ou les deux, pour donner le produit monoester ou acide dicarboxylique de formule (II) ;

ou (c) à éliminer le radical $R^{13}$, à estérifier le produit pour donner un groupe ester biolabile et à éliminer le

radical $R^{14}$ pour donner le produit de formule (II) dans lequel $R^4$ représente H et R est un groupe ester biolabile.

2. Procédé selon la revendication 1, dans lequel $R^{13}$ forme un ester N-acétyl-(1R,2S)-éphédrine et $R^{14}$ est un groupe éthyle, les groupes ester étant éliminés par hydrogénation suivie d'une hydrolyse pour donner le composé de formule (II) dans lequel R et $R^4$ représentent tous deux H.

3. Procédé selon la revendication 1, dans lequel $R^{13}$ est un groupe indanyle et $R^{14}$ est un groupe benzyle, ledit groupe benzyle étant éliminé pour donner le composé de formule (II) dans lequel R est un groupe 5-indanyle et $R^4$ représente H.

4. Procédé selon la revendication 1, dans lequel $R^{13}$ est un groupe tert-butyle et $R^{14}$ un groupe benzyle, lesdits groupes étant éliminés pour donner le composé de formule (II) dans lequel R et $R^4$ représentent tous deux H.

5. Procédé selon la revendication 1, dans lequel $R^{13}$ est un groupe tert-butyle et $R^{14}$ est un groupe benzyle, ledit groupe tert-butyle étant éliminé et le produit estérifié pour donner un groupe ester biolabile au niveau de R et le groupe benzyle est éliminé.

6. Procédé selon la revendication 1, dans lequel ledit groupe ester biolabile est un groupe :
éthyle
benzyle
1-(2,2-diéthylbutyryloxy)éthyle
2-éthylpropionyloxyméthyle
1-(2-éthylpropionyloxy)éthyle
1-(2,4-diméthylbenzoyloxy)éthyle
α-benzoyloxybenzyle
1-(benzoyloxy)éthyle
2-méthyl-1-propionyloxy-1-propyle
2,4,6-triméthylbenzoyloxyméthyle
1-(2,4,6-triméthylbenzoyloxy)éthyle
pivaloyloxyméthyle
phénéthyle
phènpropyle
2,2,2-trifluoroéthyle
1- ou 2-naphtyle
2,4-diméthylphényle
4-t-butylphényle
ou 5-indanyle.

7. Procédé selon la revendication 1, 2 ou 4 de production du composé :

Acide (S)-cis-4-{1-(2-carboxy-3-(2-méthoxyéthoxy)propyl)-1-cyclopentanecarboxamido}}1-cyclohexanecarboxylique.

8. Procédé selon la revendication 1, 3 ou 5 de production du composé :

Acide (S)-cis-4-{1-(2-(5-indanyloxycarbonyl)-3-(2-éthoxyéthoxy)propyl)-1-cyclopentanecarboxamido}-1-cyclohexanecarboxylique.

9. Composé de formule (VI) tel que défini dans la revendication 1.

10. Composé selon la revendication 9, dans lequel $R^{13}$ est un groupe 5-indanyle, tert-butyle ou forme un ester N-acétyl-(1R,2S)-éphédrine.